(19) Europäisches Patentamt

European Patent Office

Office européen des brevets

(11) **EP 1 541 123 A1**

(12) **DEMANDE DE BREVET EUROPEEN**

(43) Date de publication:
**15.06.2005 Bulletin 2005/24**

(51) Int Cl.[7]: **A61K 7/32**, A61K 7/34,
A61K 7/36

(21) Numéro de dépôt: **04292722.8**

(22) Date de dépôt: **17.11.2004**

(84) Etats contractants désignés:
**AT BE BG CH CY CZ DE DK EE ES FI FR GB GR
HU IE IS IT LI LU MC NL PL PT RO SE SI SK TR**
Etats d'extension désignés:
**AL HR LT LV MK YU**

(30) Priorité: **12.12.2003 FR 0351034**

(71) Demandeur: **L'OREAL**
**75008 Paris (FR)**

(72) Inventeurs:
• **Lemoine, Cyril**
**95380 Puiseux-en-France (FR)**
• **Beau, Nathalie**
**95610 Eragny-sur-Oise (FR)**
• **Prud'homme, Estelle**
**75018 Paris (FR)**

(74) Mandataire: **Miszputen, Laurent**
**L'OREAL - D.I.P.I.**
**25-29 Quai Aulagnier**
**92600 Asnières (FR)**

(54) **Composition cosmétique déodorante comprenant l'association du gluconate de zinc et d'un sel d'aluminium anti-transpirant**

(57) L'invention a pour objet une composition cosmétique déodorante comprenant :

a) le gluconate de zinc et
b) au moins un sel d'aluminium anti-transpirant ; le rapport en poids gluconate de zinc /sel d'aluminium allant de 1/100 à 10/1.

L'invention concerne également un procédé de traitement cosmétique de la transpiration humaine et des odeurs axillaires humaines à l'aide de cette composition.

EP 1 541 123 A1

**Description**

L'invention a pour objet une composition cosmétique déodorante comprenant au moins :

a) le gluconate de zinc et
b) au moins un sel d'aluminium anti-transpirant ; le rapport en poids gluconate de zinc /sel d'aluminium allant de 1/100 à 10/1.

L'invention concerne également un procédé de traitement cosmétique de la transpiration humaine et des odeurs axillaires humaines à l'aide de cette composition.

Dans le domaine cosmétique, il est bien connu d'utiliser en application topique, des produits déodorants contenant des substances actives de type anti-transpirant de type bactéricide ou du type absorbeur d'odeurs pour diminuer, voire supprimer, les odeurs axillaires généralement désagréables.

Les substances bactéricides inhibent le développement de la flore cutanée responsable des odeurs axillaires. Ils ont pour inconvénient de ne pas être actifs sur l'odeur de la sueur déjà développée. Parmi les produits bactéricides, le plus couramment employé est le Triclosan (2,4,4'-trichloro-2'-hydroxydiphényléther), qui présente l'inconvénient de modifier de façon importante l'écologie de la flore cutanée, d'être inhibé par certains composés, comme par exemple les tensio-actifs non-ioniques, couramment utilisés dans la formulation de compositions cosmétiques. Par ailleurs, le caractère insoluble du Triclosan dans l'eau ne permet pas son incorporation dans des formules essentiellement aqueuses.

Les substances anti-transpirantes ont pour effet de limiter le flux sudoral. Elles sont généralement constituées de sels d'aluminium. Leur efficacité déodorante est limitée lorsqu'ils sont utilisés seuls. De plus, dans des concentrations élevées, ces susbstances présentent un potentiel irritant pour la peau.

On connaît dans le brevet EP 768 080 des compositions déodorantes aqueuses en particulier des émulsions eau/silicone contenant comme actif absorbeur d'odeurs des sels de zinc hydrosolubles comme le pyrrolidone carboxylate de zinc, le sulfate de zinc, le chlorure de zinc, le lactate de zinc, le gluconate de zinc et le phénolsulfonate de zinc. Leur efficacité déodorante n'est pas encore pleinement satisfaisante lorsqu'ils sont utilisés seuls.

On connaît également dans le brevet US 6,426,061 des compositions pour combattre le développement des odeurs de la transpiration de la peau humaine comprenant l'association des actifs suivants : (1) un inhibiteur de récepteur androgène (resvératrol, epigallocatéchine-3-gallate ou acide flufénamique) ; (2) un actif anti-DHT (sels de zinc en particulier le sulfate de zinc) (3) un inhibiteur de protéines porteuses d'odeurs, (4) un sel d'aluminium anti-transpirant et (5) un agent antimicrobien du type chlorhexidine digluconate ou chlorhexidine diacétate. Ces compositions présentent l'inconvénient d'utiliser des agents antimicrobiens particulièrement actifs sur la flore cutanée.

On connaît dans la demande de brevet WO01/52804 des compositions déodorantes à base de sels antitranspirants auxquels on propose de rajouter des chélatants de métaux de transition. Ces formulations sont potentiellement écotoxiques et posent des problèmes sur l'environnement.

On connaît également dans la demande de brevet WO01/99376 des compositions déodorantes contenant des inhibiteurs d'arylsulfatase parmi lesquels sont mentionnés les sels d'aluminium et gluconate de zinc. Ce document décrit en particulier un exemple de stick anti-transpirant contenant 20% en poids d'aluminium chlorhydrate et 0,1% de gluconate de zinc. Ce type de composition produit un taux élevé de résidu blanc sur la peau après application.

Le but de la présente invention est donc de disposer de nouvelles compositions cosmétiques comprenant un système déodorant ayant une efficacité supérieure à celle des sels anti-transpirants et à celle des sels de gluconate de zinc utilisés seuls et qui ne présentent pas les inconvénients des produits déodorants de l'art antérieur tel qu'évoqués précédemment.

La demanderesse a découvert qu'une telle composition est obtenue en utilisant l'association du gluconate de zinc et d'un sel d'aluminium antitranspirant dans un rapport en poids gluconate de zinc /sel d'aluminum variant de 1/100 à 10/1.

La demanderesse a découvert de manière inattendue que cette association particulière présentait un effet de synergie au niveau de la diminution de l'intensité de l'odeur par rapport aux actifs utilisés individuellement.

Elle a également découvert que cette association particulière pouvait être formulée dans des compositions déodorantes cosmétiquement acceptables dont les teneurs en résidu visible sur la peau à l'application ou après séchage de la composition après l'application sont faibles et comparables aux produits déodorants actuellement sur le marché.

De façon plus précise, l'invention a pour objet une composition cosmétique déodorante, comprenant au moins :

a) le gluconate de zinc et
b) au moins un sel d'aluminium anti-transpirant; le rapport en poids gluconate de zinc /sel d'aluminium allant de 1/100 à 10/1 et plus préférentiellement de 1/20 à 5/1.

L'invention concerne également un procédé de traitement cosmétique de la transpiration humaine et des odeurs axillaires humaines à l'aide de cette composition.

Au sens de la présente invention, on entend par "composition déodorante" toute composition capable de réduire le flux sudoral, masquer, absorber, améliorer et/ou réduire l'odeur désagréable résultant de la décomposition de la sueur humaine par des bactéries.

Par " sel d'aluminium anti-transpirant" , on entend tout sel ou tout complexe d'aluminium ayant pour effet de diminuer ou limiter le flux sudoral.

Les sels d'aluminium conformes à l'invention sont choisis de préférence parmi les halohydrates de d'aluminium ; les halohydrates d'aluminium et de zirconium, les complexes d'hydroxychlorure de zirconium et d'hydroxychlorure d'aluminium avec un acide aminé tels que ceux décrits dans le brevet US-3792068 communément connus sous l'appellation "ZAG complexes".

Parmi les sels d'aluminium, on peut citer en particulier le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

Parmi les sels d'aluminium et de zirconium, on peut citer en particulier l'aluminium zirconium octachlorohydrate, l'aluminium zirconium pentachlorohydrate, l'aluminium zirconium tetrachlorohydrate, l'aluminium zirconium trichlorohydrate.

Les complexes d'hydroxychlorure de zirconium et d'hydroxychlorure d'aluminium avec un acide aminé sont généralement connus sous l'appellation ZAG (lorsque l'acide aminé est la glycine). Parmi ces produits on peut citer les complexes aluminium zirconium octachlorohydrex glycine, aluminium zirconium pentachlorohydrex glycine, aluminium zirconium tetrachlorohydrex glycine et aluminium zirconium trichlorohydrex glycine.

On utilisera plus particulièrement le chlorhydrate d'aluminium sous forme activée ou non activée.

Les sels d'aluminium anti-transpirants peuvent être présents dans la composition selon l'invention à raison d'environ 0,5 à 25% en poids par rapport au poids total de la composition.

Le gluconate de zinc peut être présent dans la composition selon l'invention à raison d'environ 0,1 à 10% en poids et plus préférentiellement de 0.1 à 7% en poids par rapport au poids total de la composition.

Les compositions déodorantes selon l'invention destinées à l'usage cosmétique peuvent se présenter sous forme de lotions, de crèmes ou de gels fluides distribués en spray aérosol, en flacon pompe ou en roll-on, sous forme de crèmes épaisses distribuées en tubes ou en grille ; sous forme de bâtonnets (sticks), et contenir à cet égard les ingrédients généralement utilisés dans ce type de produits et bien connus de l'homme de l'art, sous réserve qu'ils n'interfèrent pas avec le sel d'aluminium et le gluconate de zinc décrit dans la présente invention.

Les compositions déodorantes selon l'invention destinées à l'usage cosmétique peuvent comporter au moins une phase aqueuse. Elle sont notamment formulées en lotions aqueuses ou en émulsion eau-dans-huile, huile-dans-eau, ou en émulsion multiple (émulsion triple huile-dans-eau-dans-huile ou eau-dans-huile-dans-eau (de telles émulsions sont connues et décrites par exemple par C. FOX dans « Cosmetics and Toiletries » - november 1986 - Vol 101 - pages 101-112).

La phase aqueuse desdites compositions contient de l'eau et en général d'autres solvants solubles ou miscibles dans l'eau. Les solvants solubles ou miscibles dans l'eau comprennent les mono alcools à chaîne courte par exemple en $C_1$-$C_4$ comme l'éthanol, l'isopropanol ; les diols ou les polyols comme l'éthylèneglycol, le 1,2-propylèneglycol, le 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine.

Selon une forme particulière de l'invention, les compositions anti-transpirantes peuvent être anhydres.

Par "anhydre", on entend au sens de l'invention, une composition dont la teneur en eau libre ou ajoutée est inférieure à 3% et de préférence dont la teneur en eau ajoutée est inférieure à 1% en poids par rapport au poids total de la composition.

Les compositions selon l'invention comprennent de préférence au moins une phase liquide organique non-miscible dans l'eau Celle-ci comprend en général un ou plusieurs composés hydrophobes qui rendent ladite phase non-miscible dans l'eau. Ladite phase est liquide (en l'absence d'agent structurant) à température ambiante (20-25 °C). De manière préférentielle, la phase organique liquide organique non-miscible dans l'eau conforme à l'invention est généralement constituée d'une huile ou de mélange d'huiles et comprend au moins 80% de composés ayant une vapeur de pression ne dépassant pas la valeur 4 kPa (30 mm Hg) à 25°C.

La phase liquide organique non-miscible dans l'eau contient de préférence une ou plusieurs huiles émollientes siliconée ou hydrocarbonée, volatiles ou non-volatiles. Ces huiles émollientes sont notamment décrites dans les brevets US 4,822,596 et US 4,904,463.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones $D_4$, $D_5$ ou $D_6$.

Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Corning sous le nom de « Dow Corning 245 Fluid » ; les polyalkylarylsiloxanes comme par exemple les polyméthylphénylsiloxanes, commercialisés par la société Dow Corning sous le nom de « Dow Corning 556 Fluid » ; les copolymères polyéther et siloxane, comme par exemple les diméthicone copolyols.

Parmi les huiles émollientes non-volatiles utilisables dans la présente invention, on peut citer par exemple : les dérivés hydrocarbonés, les huiles minérales, les alcools gras, les esters d'alcools en $C_3$-$C_{18}$ avec des acides en $C_3$-$C_{18}$, les esters de l'acide benzoïque avec des alcools en $C_{12}$-$C_{18}$ et leurs mélanges, des polyols en $C_2$-$C_6$ choisis de préférence parmi le glycérol, le propylèneglycol ou le sorbitol, les polymères de polalkylène glycol.

Les huiles émollientes sont présentes de préférence dans des quantités allant de 1 à 50% en poids et plus préférentiellement de 5 à 40% en poids par rapport au poids total de la composition.

La composition cosmétique déodorante selon l'invention peut contenir un ou plusieurs actifs déodorants additionnels comme par exemple des agents bactériostatiques ou des agents bactéricides tels que le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,5,10-triénol (Farnesol) ; les sels d'ammonium quaternaires comme les sels de cetyltrimethylammonium, les sels de cétylpyridinium ; la chlorhexidine et les sels; le monocaprate de diglycérol, le monolaurate de diglycérol , monolaurate de glycérol ; les sels de polyhexaméthylène biguanide.

Afin d'améliorer l'homogénéité du produit, on peut utiliser en plus un ou plusieurs agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

Les agents de suspension sont présents de préférence dans des quantités allant de 0,1 à 5% en poids et plus préférentiellement de 0.2 à 2% en poids par rapport au poids total de la composition.

Les compositions selon l'invention peuvent également contenir en plus au moins une poudre organique.

Parmi les charges utilisables selon l'invention, on peut citer les poudres organiques. On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m3), 551 DE 20 (granulométrie d'environ 30 µm et masse volumique 65 kg/m3), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyllysine commercialisée sous la dénomination AMIHOPE LL-11 par la Société Ajinomoto ; les particules de microdispersion de cire, qui ont de préférence des dimensions moyennes inférieures à 1 µm et notamment allant de 0,02 µm à 1 µm, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges. La ou les poudres organiques peuvent être présentes par exemple en une quantité

La composition cosmétique selon l'invention peut comprendre en outre des adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants, des agents propulseurs ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Les cires peuvent être choisies parmi les cires animales, fossiles, végétales, minérales ou de synthèse. On peut citer notamment les cires d'abeille, les cires de Carnauba, de Candellila, de canne à sucre, du Japon, les ozokérites, la cire de Montan, les cires microcristallines, les paraffines, les cires et résines de silicone.

Les épaississants, de préférence non ioniques, peuvent être choisis parmi les gommes de guar et celluloses modifiées ou non modifiées telles que la gomme de guar hydroxypropylée, la cétylhydroxyéthylcellulose, les silices comme par exemple la Bentone Gel MIO vendue par la société NL INDUSTRIES ou la Veegum Ultra, vendue par la société POLYPLASTIC.

Les quantités de ces différents constituants pouvant être présents dans la composition cosmétique selon l'invention sont celles classiquement utilisées dans compositions déodorantes.

Les compositions selon l'invention peuvent également contenir également un ou plusieurs autres agents structurants ou gélifiants de la phase liquide organique non-miscible dans l'eau de la composition tels que les alcools gras solides linéaires et/ou les cires ; les acides gras ou leurs sels (acide stérique, stéarate de sodium, acide 12-hydroxystéarique) ; les dibenzylidene alditols (DBS) ; le lanostérol, les dérivés du N-acyl amino-acide ; les dérivés de di ou triacides carboxyliques comme les alkyl N, N'-dialkylsuccinamides (ie : dodécyl N, N'—dibutytsuccinamide) ; les organopolysiloxane élastomères tels que ceux décrits dans la demande WO97/44010

La composition selon l'invention peut encore être pressurisée et être conditionnée dans un dispositif aérosol.

La présente invention a pour objet un dispositif aérosol constitué par :

(A) un récipient comprenant une composition déodorante telle que définie précédemment,

(B) au moins un agent propulseur et un moyen de distribution de la dite composition aérosol.

Les propulseurs généralement utilisés dans ce type de produits et bien connus de l'homme de l'art, sont comme par exemple le diméthyléther (DME) ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon@ et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT. On peut également utiliser en tant qu'agent propulseur le gaz carbonique, le protoxyde d'azote, l'azote ou l'air comprimé.

La composition contenant le ou les actifs déodorants et le ou les agents propulseurs peuvent se trouver dans le même compartiment ou dans des compartiments différents dans le récipient aérosol. Selon l'invention, la concentration en agent propulseur varie généralement de 5 à 95% en poids pressurisée et plus préférentiellement de 50 à 85% en poids par rapport au poids total de la composition pressurisée.

Le moyen de distribution, qui forme une partie du dispositif aérosol, est généralement constitué par une valve de distribution commandée par une tête de distribution, elle même comprenant une buse par laquelle la composition aérosol est vaporisée. Le récipient contenant la composition pressurisée peut être opaque ou transparent. Il peut être en verre, en matériau polymérique ou en métal, recouvert éventuellement d'une couche de vernis protecteur.

La présente invention a également pour objet un procédé cosmétique pour traiter les odeurs axillaires humaines, consistant à appliquer sur la surface axillaire une quantité efficace d'une composition telle que définie ci-dessus.

Les exemples qui suivent servent à illustrer la présente invention.

**I/ Comparaison de l'activité déodorante de l'association gluconate de zinc/sel d'aluminium par rapport aux association**

Protocole du test d'efficacité déodorante triclosan/sel d'aluminium et ricinoleate de zinc /sel d'aluminium.

Des recueils de sueur axillaire sont effectués en sauna sur 6 volontaires, les échantillons de sueur individuelle, conservés dans la glace pendant quelques heures, sont alors pratiquement inodores. Ils sont ensuite mélangés et répartis en aliquots de 1 ml. Les actifs sont rajoutés à ces aliquots, puis mis à incuber à l'étuve 37°C. Après 24 heures d'incubation, un jury d'experts évalue l'intensité de l'odeur comparativement à un échantillon témoin : 1 ml de sueur incubée sans ajout d'actif. L'intensité est évaluée sur une échelle de 0 (pas d'odeur) à 4 (très forte odeur).

Les résultats sont exprimés en % de variation de l'intensité de l'odeur comparativement à cet échantillon de sueur témoin (moyenne des % de variation à T24h).

$$\Delta = \frac{\text{(intensité odeur de l'échantillon témoin - intensité odeur de l'échantillon avec actif )}}{\text{intensité odeur de l'échantillon témoin}} \times 100$$

| Actifs testés | Quantité testée (mg MA/ml de sueur | % de diminution de l'intensité de l'odeur |
|---|---|---|
| ACH | 0,1 mg MA | -37% |
| (A) | 0,5 mg MA | -19% |
| (B) | 0,4 mg MA | -49% |
| (C) | 0,3 mg MA | -4% |
| ACH + (A) | 0,6 mg MA | -83% |
| ACH + (B) | 0,5 mg MA | -46% |
| ACH + (C) | 0,4 mg MA | -38% |
| ACH : Aluminium chlorohydrate (Micro Dry - Reheis) (A) : Gluconate de zinc (Govobio G Zn - Seppic) (B) : Triclosan (CIBA) (C) : Ricinoleate de zinc (Grillo Werke) | | |

On constate que l'association gluconate de zinc/sel d'aluminium (rapport en poids : 5/1) conduit à un effet de synergie au niveau de la diminution de l'intensité de l'odeur contrairement aux associations Triclosan/sel d'aluminium et ricinoléate de zinc/sel d'aluminium.

**II/ Test de microbiologie : activité peu bactéricide du gluconate de zinc par rapport au Triclosan**

**Protocole** :

Le test décrit ici permet la détermination quantitative de l'activité bactéricide d'une composition sur des germes en conditions optimales de croissance à savoir des germes du type Corynebacterium xerosis (Collection de l'Institut Pasteur n° 5216); Staphylococcus Hominis (Collection de l'Institut Pasteur n° 81 57) et Brevibacterium Epidermidis (Collection de l'Institut Pasteur n° 102110 ) cultivés sur gélose Tryptocaséine soja en pente. La veille du test, dans un pilulier, on dépose 32 g de bouillon Tryptocaséine soja et l'on met à incuber à 35°C. Le jour du test, on ajoute 4 g de la composition à tester et on homogénéise au Vortex.

Un témoin de croissance sans produit est préparé dans les mêmes conditions afin de vérifier que les germes sont dans des conditions de croissance favorables pendant toute la durée du test.

Pour la préparation de l'inoculum, cinq jours avant le début du test, on pratique un repiquage des deux souches bactériennes sur milieu approprié. On incube 5 jours à 35°C. Le jour du test, on lave la pente avec environ 9 ml de diluant. La suspension obtenue titre à 108 germes/ml (on effectue un dénombrement). On introduit 4 ml d'inoculum dans le pilulier, ce qui correspond à un taux de 107 bactéries par gramme de préparation. On place le pilulier dans un incubateur-agitateur (35°C - 200 RPM).

Après chaque temps de contact (2, 4, 6 et 24 heures), on homogénéise le contenu du pilulier au Vortex. On effectue des dilutions décimales. On dépose en surface de boîtes de Pétri gélosée (milieu Eugon LT 100). On incube les boîtes de Pétri 6 à 7 jours à l'étuve à 35°C.

On procède au comptage des colonies sur les boîtes contenant plus de 20 et moins de 200 colonies.

| | |
|---|---|
| résultats identiques | nulle, |
| 1 Log de réduction | faible, |
| 2 Log de réduction | moyenne, |

(suite)

| 3 Log de réduction | bonne, |
|---|---|
| ≥ 4 Log de réduction | excellente. |

On a réalisé d'une part une formule 1 avec le gluconate de zinc et une formule 2 avec le Triclosan ; les supports ont été choisis de manière à être compatibles avec l'actif déodorant.

| Ingrédients | Formule 1 (invention) | Formule 2 (art antérieur) |
|---|---|---|
| Triclosan | | 0,1g |
| Gluconate de zinc | 1 g | |
| Polyéthyleneglycol à 8 OE | 16,8 g | 6g |
| Copolymère réticulé acide acrylique/acrylate d'alkyle en $C_{10}$-$C_{30}$ | - | 0,9g |
| Hydroxyde de sodium pur | - | qs (pH=7) |
| Eau désionisée microbiologiquement propre | 82,8 g | 93,0 g |

On mesure l'activité bactéricide de chacune des formulations 1 et 2 vis à vis des souches *Corynebacterium xerosis, Staphylococcus hominis, Brevibacterium epidermidis* et on l'a compare avec une formule sans actif (placebo). Les résultats obtenus sont résumés dans le tableau suivant :

| Compositions | Efficacité à 24 heures par rapport au gel sans actif | | |
|---|---|---|---|
| Souches | *Corynebacterium xerosis* (CIP 5216) | *Staphylococcus hominis* | *Brevibactérium epidermis* |
| **1 (invention)** | Nulle | Moyen | Nulle |
| **2 (art antérieur)** | Excellent | / | / |

On observe que dans la formule 1 , le gluconate de zinc utilisé à une concentration 10 fois plus élevée que celle du Triclosan a une activité antibactérienne faible ou moyen sur les différentes souches testées. Il a donc un spectre d'activité bactéricide sensiblement moins large que celui du Triclosan et respecte d'avantage la flore cutanée.

**Exemples 3 et 4** : **Sticks déodorants**

| Ingrédients | Formule 3 (selon l'exemple 1.4 de la demande WO 01/99376) | Formule 4 (invention) |
|---|---|---|
| Cyclopentasiloxane (DC245 –DOW CORNING) | 23 g | 32 g |
| Hexyldecyl stearate (Eutanol G16 S – COGNIS) | 15 g | / |
| PPG-14 Butyl ether (Ucon Fluid AP- Amerchol) | 5 g | 10 g |
| Huile de ricin hydrogénée (Cutina HR- Cognis) | 6 g | 4 g |
| Alcool cétéarylique (Lanette O- Cognis) | 8 g | / |
| Cetearyl alcohol/Ceteareth-30 80/20 (Sinnowax AO-Cognis) | 15 g | / |
| Talc | 8 g | 2 g |
| Aluminium Chlorhydrate (Micro Dry – Reheis) | 20 g | 20 g |
| Gluconate de zinc (Govobio G Zn – Seppic) | 0,1 | 1 g |
| Alcool stéaryliquel | / | 14 g |
| PEG-8 distearate (Distéarate de PEG 400- Stéarines Dubois) | / | 2 g |
| $C_{12-15}$ alkyl benzoate (Finsolv TN- Witco) | / | 15 g |
| | 100 | 100 |

On chauffe la cyclopentasiloxane à 65°C. On ajoute les autres ingrédients (1 par 1) en restant à 65-70°C. On homogénéise l'ensemble (solution transparente) pendant 15 minutes. On ajoute les 2 actifs déodorants et le talc. On refroidit à environ 55°C (quelques degrés Celcius au dessus de l'épaississement du mélange) et on coule dans les sticks. On met à 4°C pendant 30 minutes.

On mesure selon le test décrit ci-dessous le dépôt de résidu blanc des exemples 3 et 4 après application

**Protocole**

La mesure est réalisée sur un appareil de type Minolta CR300. Les produits sont appliqués de façon homogène obtenir environ 1g de produit pour 40 cm² sur un papier noir type Canson feuille mi-teinte. La mesure est réalisée immédiatement après application. Une moyenne est faite sur deux mesures.

On mesure un delta L :

$$\Delta L = L^* \text{ produit- } L^* \text{ référence}$$

L* référence du papier noir type Canson feuille mi teinte : L* référence = 19.45

On estime qu'un produit est blanchissant et non cosmétiquement acceptable pour un ΔL supérieur ou égal à 35.

Les résultats obtenus sont résumés dans le tableau suivant :

| Composition | ΔL |
|---|---|
| 3 (art antérieur) | 35 |
| 4 (invention) | 5 |

On constate que la composition 3 selon l'art antérieur contenant l'association gluconate de zinc / chlorhydrate d'aluminium dans un rapport 1/200 produit un taux élevé de résidu blanc sur le substrat tandis que la composition 4 selon l'invention produit un résidu blanc très faible comparable aux sticks déodorants actuellement sur le marché tels que les produits commerciaux "Lady Speed Stick - Clean Glide" de Colgate ou " Secret Clear Dry"de (Procter & Gamble).

Exemple 5 : Roll-on (émulsion)

| Phase | Ingrédients | Formule 5 |
|---|---|---|
| A | Aluminium Chlorhydrate (50%solution) (Chlorhydrol 50% USP) | 40 g |
| | Gluconate de zinc (Govobio G Zn – Seppic) | 2 g |
| B | Steareth-21 (Brij 721-ICI) | 2g |
| | Steareth-2 (Brij 2 – ICI) | 2g |
| | Steareth-5 Stearate | 1g |
| | PPG-15 stearyl ether (Arlamol E – ICI) | 1,5g |
| | Cyclopentasiloxane (DC245 –DOW CORNING) | 3,5g |
| C | Eau | 48g |
| | | 100g |

On chauffe les phases (B) et (C) séparément à 70°C. On mélange (B) et (C) sous agitation Turax 5min puis refroidir à 55°C sous pâle. On ajoute A doucement en agitant. On homogénéise 1 à 3 minutes. On refroidit à 35°C sous agitation. La formule est stable 2 mois à 45°C.

On mesure le taux de résidu blanc selon le même test décrit dans les exemples 3 et 4. On obtient un ΔL égal à 2.

**Exemple 6 : Spray non aérosol (Emulsion obtenue par inversion de phase)**

| Ingrédients | Formule 6 |
|---|---|
| Aluminium Chlorhydrate (50%solution) (Chlorhydrol 50% USP) | 20g |
| Gluconate de zinc (Govobio G Zn – Seppic) | 3g |
| Cetearyl isononanoate (and) Cetearyl alcohol (and) Ceteareth-20 (and) Glycerin (and) Glyceryl stearate (and) Ceteareth-12 (and) Cetyl palmitate (Emulgade CM- Cognis) | 15g |
| Eau | 62g |
| | 100g |

On solubilise le gluconate dans l'eau et ajouter à l'Emulgade CM sous agitation modérée. On ajoute la solution de sel d'aluminium sous agitation modérée. La formule est stable 2 mois à 45°C. On mesure le taux de résidu blanc selon le même test décrit dans les exemples 3 et 4. On obtient un $\Delta L$ égal à 0.

**Exemple 7 : Aérosol**

| Ingrédients | Formule 7 |
|---|---|
| Stearalkonium Bentonite (Tixogel MP 250 – Sud Chemie Rheologicals United Catalysts Inc.) | 0.5g |
| Aluminium Chlorhydrate (Micro Dry- Reheis) | 7g |
| Gluconate de zinc (Govobio G Zn – Seppic) | 0.5g |
| $C_{12-15}$ alkyl benzoate (Finsolv TN- Witco) | 3g |
| Isobutane | 80g |
| Tri Ethyl Citrate (Citroflex-Morflex) | 1g |
| Palmitate d'isopropyle | 1g |
| Cyclopentasiloxane (DC245 –DOW CORNING) | 7g |
| | 100g |

On introduit les solvants et l'argile modifiée hydrophobe puis on agite au Turax jusqu'à homogénéisation. On ajoute ensuite le sel d'aluminium (anti-transpirant) et le gluconate de zinc tout en agitant. Le propulseur est ensuite introduit de manière classique. On mesure le taux de résidu blanc selon le même test décrit dans les exemples 3 et 4. On obtient un ∆L égal à 4 pour la formule 7.

**Revendications**

1.  Composition cosmétique déodorante comprenant au moins :

    a) le gluconate de zinc et
    b) au moins un sel d'aluminium anti-transpirant; le rapport en poids gluconate de zinc /sel d'aluminium allant de 1/100 à 10/1.

2.  Composition selon la revendication 1, où le rapport en poids gluconate de zinc /sel d'aluminium varie de 1/20 à 5/1.

3.  Composition selon la revendication 1 ou 2, où le sel anti-transpirant est choisi parmi les halohydrates d'aluminium ; les halohydrates d'aluminium et de zirconium ; les complexes d'hydroxychlorure de zirconium et d'hydroxychlorure d'aluminium avec un acide aminé.

4.  Composition selon la revendication 3, où le sel d'aluminium anti-transpirant est choisi parmi le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

5.  Composition selon la revendication 3, où le sel d'aluminium anti-transpirant est choisi parmi l'aluminium zirconium octachlorohydrate, l'aluminium zirconium pentachlorohydrate, l'aluminium zirconium tetrachlorohydrate, l'aluminium zirconium trichlorohydrate .

6.  Composition selon la revendication 3, où le sel d'aluminium anti-transpirant est choisi parmi les complexes d'hydroxychlorure de zirconium et d'hydroxychlorure d'aluminium avec la glycine.

7.  Composition selon la revendication 6, où le sel d'aluminium anti-transpirant est choisi parmi les complexes aluminium zirconium octachlorohydrex glycine, aluminium zirconium pentachlorohydrex glycine, aluminium zirconium tetrachlorohydrex glycine et aluminium zirconium trichlorohydrex glycine.

8.  Composition selon l'une quelconque des revendications précédentes, où le sel d'aluminium anti-transpirant est le chlorhydrate d'aluminium sous forme activée ou non activée.

9.  Composition selon l'une quelconque des revendications 1 à 8, où le ou les sels d'aluminium anti-transpirants sont présents dans des quantités allant de 0,5 à 25% en poids par rapport au poids total de la composition.

10. Composition selon l'une quelconque des revendications 1 à 9, où le gluconate de zinc peut être présent dans des quantités allant de 0,1 à 10% en poids et plus préférentiellement de 0,1 à 7% en poids par rapport au poids total de la composition.

11. Composition selon l'une quelconque des revendications 1 à 10, **caractérisée par le fait qu'**elle se présente sous forme de lotion, de crème ou de gel fluide distribué en spray aérosol, en flacon pompe ou en roll-on ; sous forme de crème ou gel distribué en tube ou en grille ; sous forme de bâtonnet (stick).

12. Composition selon l'une quelconque des revendications précédentes, **caractérisée par le fait qu'**elle comprend au moins une phase aqueuse.

13. Composition selon la revendication 12 , **caractérisée par le fait qu'**elle se présente sous forme de lotion aqueuse , sous forme d'émulsion eau-dans-huile, huile-dans-eau ; sous forme d'émulsion multiple.

14. Composition selon la revendication 13, où la phase aqueuse contient de l'eau et un ou plusieurs solvants solubles ou miscibles dans l'eau.

**15.** Composition selon la revendication 13, où les solvants solubles ou miscibles dans l'eau sont choisis parmi les mono alcools en $C_1$-$C_4$; ; les diols ; les polyols.

**16.** Composition selon l'une quelconque des revendications 1 à 11, **caractérisée par le fait qu'**elle est anhydre.

**17.** Composition selon l'une quelconque des revendications 1 à 16, **caractérisée par le fait qu'**elle comprend au moins une phase liquide organique non-miscible dans l'eau.

**18.** Composition selon la revendication 17, dans laquelle la phase liquide organique comprend une ou plusieurs huiles émollientes siliconée ou hydrocarbonée, volatiles ou non-volatiles.

**19.** Composition selon la revendication 18, dans laquelle les huiles émollientes sont présentes dans des quantités allant de 1 à 50% en poids et plus préférentiellement de 5 à 40% en poids par rapport au poids total de la composition.

**20.** Composition selon l'une quelconque des revendications 1 à 16, **caractérisée par le fait qu'**elle comprend en plus un ou plusieurs actifs déodorants additionnels.

**21.** Composition selon la revendication 20, **caractérisée par le fait qu'**elle comprend en plus un ou plusieurs agents bactériostatiques ou agents bactéricides.

**22.** Composition selon l'une quelconque des revendications 1 à 21, **caractérisée par le fait qu'**elle comprend en plus un ou plusieurs agents de suspension.

**23.** Composition selon l'une quelconque des revendications 1 à 22, **caractérisée par le fait qu'**elle comprend en plus au moins une poudre organique.

**24.** Composition selon l'une quelconque des revendications 1 à 22, **caractérisée par le fait qu'**elle comprend en plus au moins un additif cosmétique choisi parmi les cires, les adoucissants, les antioxydants, les opacifiants, les stabilisants, les agents hydratants, les vitamines, les parfums, des bactéricides, les conservateurs, les polymères, les parfums, les agents épaississants, des agents propulseurs.

**25.** Composition selon l'une quelconque des revendications 17 à 24, **caractérisée par le fait qu'**elle comprend en plus un ou plusieurs agents structurants ou gélifiants de la phase liquide organique non-miscible dans l'eau.

**26.** Dispositif aérosol constitué par :

(A) un récipient comprenant une composition déodorante telle que définie selon l'une quelconque des revendications 1 à 25 ;
(B) au moins un agent propulseur et un moyen de distribution de la dite composition aérosol.

**27.** Procédé cosmétique pour traiter les odeurs axillaires humaines, consistant à appliquer sur la surface axillaire une quantité efficace d'une composition telle que définie telle que définie selon l'une quelconque des revendications 1 à 25.

**28.** Utilisation de l'association du gluconate de zinc et d'un sel d'aluminium anti-transpirant dans un rapport en poids allant de 1/100 à 10/1 comme actif déodorant dans une composition cosmétique.

**EP 1 541 123 A1**

<table>
<tr><td colspan="3">Office européen<br>des brevets</td><td colspan="2">**RAPPORT DE RECHERCHE EUROPEENNE**</td><td>Numéro de la demande<br>EP 04 29 2722</td></tr>
</table>

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 6 403 067 B1 (HILLIARD JR PETER ET AL) 11 juin 2002 (2002-06-11)<br>* revendication 1 *<br>* colonne 8, ligne 7 - colonne 9, ligne 11 *<br>* colonne 17, ligne 1-13 *<br>----- | 1-28 | A61K7/32<br>A61K7/34<br>A61K7/36 |
| X | DE 101 37 901 A (HENKEL KGAA) 5 décembre 2002 (2002-12-05)<br>* page 10; exemples 1.4,1.5 * | 1-28 | |
| Y | * page 11 *<br>----- | 26 | |
| D,Y | EP 0 768 080 A (OREAL) 16 avril 1997 (1997-04-16)<br>* revendication 5 *<br>* page 5; exemple 1 *<br>----- | 1-28 | |
| Y | WO 00/68369 A (BIOSPHERICS INC) 16 novembre 2000 (2000-11-16)<br>* revendications 1,2,4,9,11,15 *<br>* page 11; exemple 2 *<br>* pages 12-15; exemples 4,6 *<br>----- | 1-28 | **DOMAINES TECHNIQUES RECHERCHES (Int.Cl.7)**<br><br>A61K |
| A | FR 2 815 856 A (OREAL) 3 mai 2002 (2002-05-03)<br>* page 5, ligne 15-31 *<br>----- | 1-28 | |
| A | US 5 643 559 A (EIGEN EDWARD ET AL) 1 juillet 1997 (1997-07-01)<br>* colonne 9, ligne 47 - colonne 10, ligne 25 *<br>* revendication 1 *<br>* colonne 17, ligne 55-59 *<br>----- | 1-28 | |

Le présent rapport a été établi pour toutes les revendications

| Lieu de la recherche | Date d'achèvement de la recherche | Examinateur |
|---|---|---|
| Munich | 30 mars 2005 | Grillenberger, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

     ...........................................................................................

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 04 29 2722

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

30-03-2005

| Document brevet cité au rapport de recherche | | Date de publication | Membre(s) de la famille de brevet(s) | | Date de publication |
|---|---|---|---|---|---|
| US 6403067 | B1 | 11-06-2002 | AU | 6321001 A | 03-12-2001 |
| | | | BR | 0110981 A | 08-04-2003 |
| | | | CA | 2409239 A1 | 29-11-2001 |
| | | | EP | 1286650 A2 | 05-03-2003 |
| | | | HU | 0302224 A2 | 28-10-2003 |
| | | | MX | PA02011404 A | 25-04-2003 |
| | | | NZ | 522697 A | 24-09-2004 |
| | | | WO | 0189452 A2 | 29-11-2001 |
| | | | US | 2002164296 A1 | 07-11-2002 |
| | | | ZA | 200209451 A | 20-11-2003 |
| DE 10137901 | A | 05-12-2002 | DE | 10137901 A1 | 05-12-2002 |
| | | | AU | 9554001 A | 02-01-2002 |
| | | | WO | 0199376 A2 | 27-12-2001 |
| | | | EP | 1393518 A2 | 03-03-2004 |
| EP 0768080 | A | 16-04-1997 | FR | 2739775 A1 | 18-04-1997 |
| | | | AT | 167627 T | 15-07-1998 |
| | | | BR | 9607243 A | 30-12-1997 |
| | | | CA | 2208243 A1 | 24-04-1997 |
| | | | CN | 1169111 A ,C | 31-12-1997 |
| | | | DE | 69600375 D1 | 30-07-1998 |
| | | | DE | 69600375 T2 | 29-10-1998 |
| | | | EP | 0768080 A1 | 16-04-1997 |
| | | | ES | 2120275 T3 | 16-10-1998 |
| | | | WO | 9714399 A1 | 24-04-1997 |
| | | | HU | 9702391 A2 | 28-05-1998 |
| | | | JP | 3006892 B2 | 07-02-2000 |
| | | | JP | 10500707 T | 20-01-1998 |
| | | | KR | 230830 B1 | 15-11-1999 |
| | | | PL | 320421 A1 | 29-09-1997 |
| | | | RU | 2141309 C1 | 20-11-1999 |
| | | | US | 6346238 B1 | 12-02-2002 |
| | | | US | 2002127193 A1 | 12-09-2002 |
| WO 0068369 | A | 16-11-2000 | US | 6585964 B1 | 01-07-2003 |
| | | | AU | 4068400 A | 21-11-2000 |
| | | | CA | 2372612 A1 | 16-11-2000 |
| | | | EP | 1173558 A1 | 23-01-2002 |
| | | | JP | 2003519246 T | 17-06-2003 |
| | | | WO | 0068369 A1 | 16-11-2000 |
| FR 2815856 | A | 03-05-2002 | FR | 2815856 A1 | 03-05-2002 |
| | | | AU | 1064602 A | 06-05-2002 |
| | | | WO | 0234222 A1 | 02-05-2002 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE
RELATIF A LA DEMANDE DE BREVET EUROPEEN NO.**

EP 04 29 2722

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

30-03-2005

| Document brevet cité au rapport de recherche | Date de publication | Membre(s) de la famille de brevet(s) | Date de publication |
|---|---|---|---|
| US 5643559 A | 01-07-1997 | US 5676937 A | 14-10-1997 |

Pour tout renseignement concernant cette annexe : voir Journal Officiel de l'Office européen des brevets, No.12/82

EPO FORM P0460